(19) Europäisches Patentamt
European Patent Office
Office européen des brevets

(11) EP 4 417 173 A1

(12) **EUROPEAN PATENT APPLICATION**

(43) Date of publication:
**21.08.2024 Bulletin 2024/34**

(21) Application number: **23156655.5**

(22) Date of filing: **14.02.2023**

(51) International Patent Classification (IPC):
**A61F 13/47** (2006.01)    **A61F 13/49** (2006.01)
**A61F 13/551** (2006.01)

(52) Cooperative Patent Classification (CPC):
**A61F 13/55145; A61F 13/47; A61F 13/49**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Ontex BV**
  **9255 Buggenhout (BE)**

• **Ontex Group NV**
  **9320 Erembodegem (BE)**

(72) Inventors:
• **MATSUO, Maria Renata Vieira**
  **3232 Quadra 9 Lote 2, 08790-420 (BR)**
• **VAZQUEZ ARANA, Mauricio**
  **30097 Duluth, Georgia (US)**

(74) Representative: **Macchetta, Andrea**
**Ontex BV**
**Korte Keppestraat 21**
**9320 Erembodegem-Aalst (BE)**

(54) **ECONOMICAL DISPOSABLE ABSORBENT ARTICLE**

(57)    Disclosed herein is a band of disposable absorbent assemblies including adjacent first and second absorbent assemblies that are connected by at least one layer of material, wherein the band comprises a weakened division line disposed between adjacent first and second absorbent assemblies, and the band of disposable absorbent assemblies is rolled or spooled into a cylindrical configuration.

Fig. 1a

**Description**

**TECHNICAL FIELD**

The invention pertains to the technical field of absorbent hygiene products. In particular, the present invention relates to disposable absorbent assemblies that comprise an upper fluid-permeable layer, a lower fluid-impermeable layer and an absorbent core interposed between them, said disposable absorbent assemblies being manufactured, distributed, and stored in an economical way.

**BACKGROUND**

Measures for lowering costs in the manufacture, distribution and storage of disposable absorbent articles have been widely explored. Manufacturers often look after measures such as reducing the basis weight of the raw materials or even removing some raw materials considered less essential, they also look for alternatives to reduce the package volume for improving distribution and storage of the disposable absorbent articles. Although many measures have been taken, there is a need for an improvement of this aspects within the disposable absorbent articles industry.

It is also necessary to look after solutions that are not only economical in the sense of monetary costs reduction, but also that reduce the environment impact of disposable absorbent products.

**SUMMARY**

The present disclosure relates to a band of disposable absorbent assemblies having at least first and second disposable absorbent assemblies, wherein each of the first and second disposable absorbent assemblies comprises an absorbent core interposed between an upper fluid-permeable layer and a lower fluid-impermeable layer. The first and the second disposable absorbent assemblies are connected by at least one layer of material; and a weakened division line is disposed between the absorbent core of the first disposable absorbent assembly and the absorbent core of the second disposable absorbent assembly. The band of disposable absorbent assemblies is rolled into a cylindrical shape.

In one aspect, at least one of the upper fluid-permeable layer and the lower fluid-impermeable layer has a compression set of 80% or less, according to the Compression test described herein.

In another aspect, each of the at least first or second disposable absorbent assemblies comprises at least 10% by weight of biobased material.

Further beneficial features are as indicated in the detailed description and dependent claims.

**DESCRIPTION OF THE DRAWINGS**

Fig.1a is a partial plan view of an exemplary, non-limiting embodiment of a band of absorbent assemblies in accordance with the present embodiments.

FIG. 1b is a cross-sectional view of an exemplary, non-limiting embodiment of a band of absorbent assemblies.

FIG. 2 is a partial plain view of an exemplary, non-limiting embodiment of an absorbent assembly in accordance with the present embodiments.

Fig. 3 is a cross-sectional view of the X-X section of an absorbent assembly of Fig. 2.

Fig. 4 is a partial plain view of an exemplary, non-limiting embodiment of a taped diaper in accordance with the present embodiments.

Fig. 5 is a cross-sectional view of the X-X section of the taped diaper of Fig. 4.

Fig. 6a is a plain view of an exemplary, non-limiting embodiment of a sanitary napkin as seen from its upper surface in accordance with the present embodiments.

Fig. 6b is a plain view of an exemplary, non-limiting embodiment of a sanitary napkin as seen from its lower surface in accordance with the present embodiments.

Fig. 7a is a plain view of an exemplary, non-limiting embodiment of an absorbent insert as seen from its upper surface in accordance with the present embodiments.

Fig. 7b is a plain view of an exemplary, non-limiting embodiment of an absorbent insert as seen from its lower surface in accordance with the present embodiments.

Fig. 8a-8d are examples of the weakened line in the band of absorbent assemblies in accordance with the present embodiments.

Fig. 8e is an example of the more than one weakened line configuration in the band of absorbent assemblies in accordance with the present embodiments.

Fig. 8f-8g are examples of the reinforcing means in the band of absorbent assemblies in accordance with the present embodiments.

Fig. 8h-8i are examples of the separation aids in the band of absorbent assemblies in accordance with the present embodiments.

Fig. 8j is an example of the visual aid in the band of absorbent assemblies in accordance with the present embodiments.

FIG. 9 is a plain view of an exemplary, non-limiting embodiment of a band of absorbent assemblies being rolled in a cylindrical shape.

FIG. 10 is an exemplary, non-limiting embodiment of a band of absorbent assemblies disposed in a cylindrical shape.

Fig. 11 is an exemplary, non-limiting embodiment of a packaged band of absorbent assemblies disposed in a cylindrical shaped.

Fig. 12 is an exemplary, non-limiting embodiment of a packaged band of absorbent assemblies disposed in a cylindrical shaped.

Fig. 13 is an exemplary, non-limiting embodiment of a packaged band of absorbent assemblies disposed in a cylindrical shaped.

Fig. 14 is an exemplary, non-limiting embodiment of a packaged band of absorbent assemblies disposed in a cylindrical shaped.

## DETAILED DESCRIPTION

Unless otherwise defined, all terms and phrases used in describing the various embodiments, including technical and scientific terms, have the meaning as commonly understood by one of ordinary skill in the art to which this invention belongs. By means of further guidance, definitions of certain terms and phrases are included to better appreciate the teaching of the present invention.

As used herein, the following terms and phrases have the following meanings:
"A", "an", and "the" as used herein refers to both singular and plural referents unless the context clearly dictates otherwise. By way of example, "a compartment" refers to one compartment, or more than one compartment.

"Comprise", "comprising", "comprises" and "comprised of" as used herein are used interchangeably with "include", "including", "includes" and/or "contain", "containing", "contains" and like terms, and are inclusive or open-ended terms that are intended to be interpreted as specifying the presence of what follows, e.g., component features, element, members, steps, and do not exclude or preclude the presence of additional, non-recited components, features, element, members, steps, or groups thereof, whether known in the art or disclosed therein. Each of these terms encompasses examples and aspects encompassed by the terms "consisting of" and "consisting essentially of'. Similarly, the term "consisting essentially of" is intended to include examples encompassed by the term "consisting of."

"Absorbent article" generally refers to devices that absorb and contain liquid, and more specifically, refers to devices that are placed against or in proximity to the body of the wearer to absorb and contain the various exudates discharged from the body. In the various embodiments described herein, the expression "absorbent article" or "personal hygiene article" or "personal hygiene absorbent article", refers to articles which are worn by females adjacent to the pudendal region which are intended to absorb and contain the various exudates which are discharged from the body (e.g., blood, menses, and urine) and is tended to include catamenial absorbent articles, incontinence pads and pantyliners.

Absorbent articles comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn.

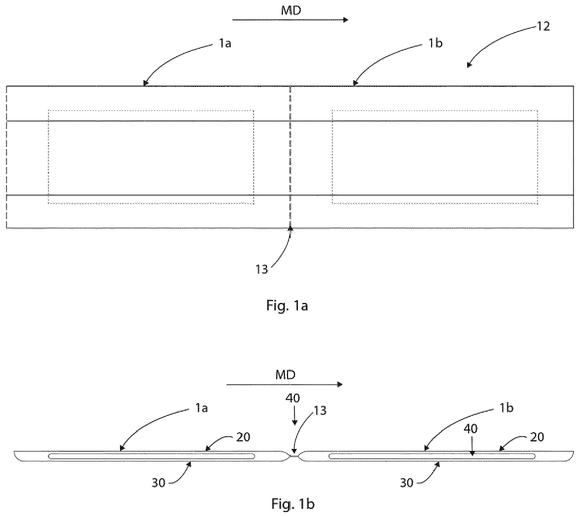

The expression "extended form" means the form of an article when it is in its laid-flat and fully stretched condition, for example, as illustrated in FIG. 1 herein.

The expression "body-facing " with respect to a side or a surface refers to that side or surface of an absorbent article and/or a component thereof that is oriented toward the body of the wearer when the article is in use. In comparison, a "garment-facing" side or surface refers to that side or surface of an absorbent article and/or a component thereof that is oriented away from the body of the wearer when the article is in use. Generally speaking, the body-facing side and the garment-facing side of a component are opposed, i.e., the opposite surfaces of the absorbent articles and/or their component members. Absorbent articles and components thereof, including the upper fluid-permeable layer, lower fluid-impermeable layer, absorbent core, and any individual layers of their components, each have a body-facing side or surface and garment-facing side or surface. In addition, layers or components of the absorbent article may be referred to as "upper" which indicates its relative location being in the direction of the body-facing side or surface, or "lower" which indicates its relative location being in the direction of the garment-facing side or surface.

The "upper fluid-permeable layer" or "top sheet" is a layer disposed on or near the body-facing side of the absorbent articles described herein, and is intended to be permeable to the fluids that are absorbed and stored by the absorbent article. According to the embodiments described herein, the upper fluid-permeable layer is a non-woven substrate having at least one layer designed to permit the passage of liquids such as menses toward the absorbent core. The materials may be left as a continuous layer or may be modified to three-dimensional, embossed, perforated webs or any other combination thereof. It may be bonded or associated to the lower fluid-impermeable layer using any known method which does not leave any hard or uncomfortable residues that would annoy the individual using the absorbent article.

"Nonwoven" refers to a manufactured sheet, web, or batt directionally or randomly oriented fibers bonded by friction and/or cohesion and/or adhesion, excluding paper and products that are woven, knitted, tufted stitch bonded incorporating binding yarns or filaments, or felted by wet milling. The fibers may be of natural or man-made origin. They may also be staple or continuous filaments or be formed in situ.

The "lower fluid-impermeable layer", "baffle" or "back sheet" are used interchangeably herein and refer to a layer or layers on or near the garment-facing side of the absorbent articles describe herein, and are intended to be substantially impermeable to the liquids that are held or stored by the absorbent article. According to some embodiments, the lower fluid-impermeable layer may be configured to permit the passage of air or vapor out of the absorbent article while blocking the passage of body fluid. In some embodiments, the fluid-impermeable layer or back sheet can be configured to block the passage of vapor as well as fluids, if desired. A fluid-impermeable layer can be made from any material or combination of materials having these properties. A suitable material is a polymeric film, such as a film comprising or consisting of a polyolefin, such as a polyethylene or a polypropylene. In some embodiments, the fluid-impermeable layer or backsheet can comprise two or more materials (e.g., a film and a nonwoven) that are operably connected to provide the desired properties.

The "absorbent medium", "absorbent core" or "absorbent body" in the absorbent article is disposed between an upper fluid-permeable layer and a lower fluid-impermeable layer and is capable of absorbing and retaining body exudates. The absorbent core may be manufactured in a wide variety of shapes (for example, rectangular, trapezoidal, T-shape, I-shape, hourglass shape, dog bone shape, etc.) and may comprise a wide variety of absorbent materials. Examples of absorbent materials include fluff pulp, cellulosic fluff pulp, tissue layers, highly absorbent polymers (so called superabsorbent polymer particles or "SAP"), absorbent foam materials, absorbent nonwoven materials and the like, or any combination of two or more of these absorbent materials. The absorbent core may comprise one or more layers of absorbent material stacked on top of each other.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present disclosure can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present disclosure. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

As used herein "eco-friendly", in relation to products, materials, or components thereof, refers to materials, or products or components thereof that are not harmful to the environment or are less harmful to the environment. Eco-friendly materials can include one or more sustainable or renewable components, such as bio-based, regenerated, biodegradable, compostable, renewable, reusable, recyclable, or recycled polymers or materials (e.g., by mechanical or chemical means), or combinations thereof. For example, natural materials may include cotton, bamboo, hemp, bast fibers, flex, jute, kenaf, or other similar fibers. Regenerated materials may include viscose, rayon, lyocell, or a combination thereof. Biodegradable materials may include biodegradable polyesters such as polylactic acid (PLA), polyhydroxy alkenoate (PHA), polyhydroxy butyrate (PHB), polybutylene adipate co-terephthalate (PBAT), polybutylene succinate (PBS), polycaprolactone (PCL), or polyvinyl alcohol (PVOH), or the like. Eco-friendly materials also include starches such as thermoplastic starch, or derived from potato, maize, corn, or the like. An eco-friendly material may comprise a mixture or blend of components, including at least one eco-friendly component. An eco-friendly component can be made entirely of an eco-friendly material or only partially of an eco-friendly material, for example a multicomponent fiber comprising polylactic acid and polypropylene in a core-sheath configuration.

As used herein, "bio-based" in relation to products, materials, or components thereof, refers to renewable products, materials, or components that mainly consist of a substance derived from living matter.

The recitation of numerical ranges by endpoints includes all numbers and fractions subsumed within that range, as well as the recited endpoints. For example, where the stated range includes one or both of the limits, ranges excluding either or both of those included limits are also included in the disclosure, e.g. the phrase "x to y" includes the range from 'x' to 'y' as well as the range greater than 'x' and less than 'y'. The range can also be expressed as an upper limit, e.g. 'about x, y, z, or less' and should be interpreted to include the specific ranges of 'about x', 'about y', and 'about z' as well as the ranges of 'less than x', less than y', and 'less than z'. Likewise, the phrase 'about x, y, z, or greater' should be interpreted to include the specific ranges of 'about x', 'about y', and 'about z' as well as the ranges of 'greater than x', greater than y', and 'greater than z'. In addition, the phrase "about 'x' to 'y'", where 'x' and 'y' are numerical values, includes "about 'x' to about 'y'".

As used herein, the terms "about," "approximate," "at or about," and "substantially" mean that the amount or value in question can be the exact value or a value that provides equivalent results or effects as recited in the claims or taught herein. That is, it is understood that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art such that equivalent results or effects are obtained. In some circumstances, the value that provides equivalent results or effects cannot be reasonably determined. In such cases, it is generally understood, as used herein, that "about" and "at or about" mean the nominal value indicated $\pm 10\%$ variation unless otherwise indicated or inferred. In general, an amount, size, formulation, parameter or other quantity or characteristic is "about," "approximate," or "at or about" whether or not expressly stated to be such. It is understood that where "about," "approximate," or "at or about" is used before a quantitative value, the parameter also includes the specific quantitative value itself, unless specifically stated otherwise.

As used herein, the terms "optional" or "optionally" means that the subsequently described component, event or circumstance can or cannot occur, and that the description includes instances where said component, event or circumstance occurs and instances where it does not.

The following description is intended to convey a thorough understanding of the embodiments by providing a number of specific embodiments and details involving an absorbent article. It is understood, however, that the invention is not limited to these specific embodiments and details, which are exemplary only. It is further understood that one possessing ordinary skill in the art, in light of known devices, systems and methods, would appreciate the use of the invention for its intended purposes and benefits in any number of alternative embodiments

Generally speaking, the invention relates to a band comprising a plurality of adjacent absorbent assemblies that are separated by weakened division lines. The embodiments described herein provide an improvement over conventional disposable absorbent articles in the sense of their economical benefit during its manufacture, distribution, and storage.

Fig. 1a shows a partial plan view of an exemplary, non-limiting embodiment of a band of disposable absorbent assemblies (12) in extended form. Generally speaking, the band of disposable absorbent assemblies (12) of the various embodiments described herein, has a plurality of absorbent assemblies, such as at least a first absorbent assembly (1a) and a second absorbent assembly (1b). The disposable absorbent assemblies (1a, 1b) are adjacent along the longitudinal direction of the band and are connected between each other through at least one layer of material, as seen in Figs. 1a-1b. Weakened division lines (13) are disposed between adjacent absorbent assemblies (e.g., 1a, 1b) for the purpose of separating one or more of the absorbent assemblies (e.g., 1a, 1b) from the band (12). While certain embodiments may be described with reference to a first and second absorbent assembly (1a, 1b), it will be understood that the band (12) of absorbent assemblies is not so limited and may include more absorbent assemblies (e.g., up to "n" absorbent assemblies) as necessary or desired, without departing from the scope and spirit of the invention.

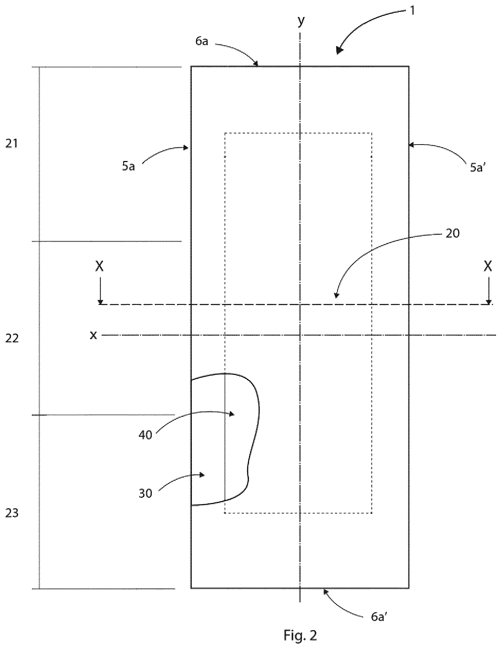

According to the various embodiments, each absorbent assembly (1a, 1b) within the band (12) comprises at least an absorbent core (40) interposed between an upper fluid-permeable layer (20) and a lower fluid-impermeable layer (30), as seen in Figs. 2-3.

The upper fluid-permeable layer or topsheet (20) may be a layer or layers made of non-woven, film, perforated film, or combinations thereof. The upper fluid-permeable layer (20) may comprise one or more natural or synthetic materials or a combination thereof. For example, it may include one or more synthetic polymers such as polyethylene, polypropylene, polyethylene terephthalate (PET) or the like. The upper fluid-permeable layer 20 or layers may comprise one or more eco-friendly materials such as cotton, bamboo, hemp, polylactic acid (PLA), polyhydroxy alkenoate (PHA), polyhydroxy butyrate (PHB), or the like. In further embodiments, the upper fluid-permeable layer may comprise a combination of synthetic polymers and eco-friendly materials, for example multicomponent fibers comprising polylactic acid and polypropylene in a core-sheath configuration. The nonwoven technology may be spubonded, meltblown, stitchbond, needlepunch, spunlace or the like. For example, in embodiments where the upper fluid-permeable layer (20) comprises a nonwoven layer, the nonwoven technology may be spunlace comprising synthetic fibers such as polyester or polypropylene so as to assure there is a sufficient compression recovery of the absorbent assembly.

The lower fluid-impermeable layer or backsheet (30) may be a layer or layers made of nonwoven, film, or combinations thereof. The lower fluid-impermeable layer (30) may comprise one or more natural or synthetic materials or a combination thereof. For example, it may include one or more synthetic polymers such as polyethylene, polypropylene, polyethylene terephthalate (PET) or the like. The lower fluid-impermeable layer (30) may comprise eco-friendly materials such as cotton, bamboo, hemp, polylactic acid (PLA), polyhydroxy alkenoate (PHA), polyhydroxy butyrate (PHB) or the like. In further embodiments, the lower fluid-impermeable layer (30) may comprise a combination of synthetic polymers and eco-friendly materials, for example in multicomponent fibers comprising polylactic acid and polypropylene in a core-sheath configuration. In some embodiments, the lower fluid-impermeable layers may be a laminate of breathable film and nonwoven layer bonded together by means of adhesive, ultrasonic bonding or thermal bonding. The nonwoven technology may be spubonded, meltblown, stitchbond, needlepunch, spunlace or the like. For example, in embodiments where the lower fluid-impermeable layer (30) comprises a nonwoven layer, the nonwoven technology may be spunlace comprising synthetic fibers such as polyester or polypropylene so as to assure there is a sufficient compression recovery of the absorbent assembly.

The absorbent core (40) may comprise superabsorbent fibers, superabsorbent particles, superabsorbent gelling materials, cellulosic fibers, foams, synthetic absorbent fibers, airlaid fibers or combinations thereof. In an embodiment, the absorbent core (40) may be a pre-formed core (e.g. absorbent core comprising airlaid fibers with SAP particles dispersed therein or absorbent cores comprising HIPE foam). In an alternative embodiment, the absorbent core (40) may be a fluff/SAP mixture (e.g. an absorbent core with an homogeneous fluff/SAP mixture or an absorbent core comprising an heterogeneous fluff/SAP mixture in pad plane or in thickness of the pad plane). The superabsorbent materials such as fibers, particles or gelling materials may come from renewable resources such as starch, polylactic acid, polysaccharide acids or the like. In an embodiment, the absorbent core (40) may comprise, among other components, a mixture of two different SAPs, wherein at least one SAP is eco-friendly. The cellulosic fibers may come from renewable resources such as softwood, hardwood, cotton, bamboo, or the like, including combinations thereof. The foamed structures may come from renewable resources such as fungus, cellulose, polylactic acid or the like. In further embodiments, the foamed structures may comprise mixtures of synthetic polymers and renewable resources. In further embodiments, the foamed structures may comprise recycled materials such as recycled polyethylene terephthalate. The synthetic absorbent fibers may be absorbent or superabsorbent polymers commonly used in disposable absorbent articles, including renewable polymers. In one exemplary embodiment the absorbent core (40) may comprise superabsorbent starch particles located within a layer of superabsorbent fibers.

In an embodiment, the absorbent core (40) may comprise one or more channels, said channels may be embossed or substantially free of absorbent material. The channels may be longitudinally or transversely oriented or a combination thereof. For example, for channels that may be substantially free of absorbent material, the channels may be formed when the upper core wrap is adhered to the lower core wrap in the regions where the absorbent core is substantially free of absorbent material. The upper and lower core wrap adhesion to form said channels may be permanent or temporary.

Referring to FIG. 2, each absorbent assembly (1) comprises a longitudinal axis "y", and a transverse axis "x". In various embodiments the longitudinal axis "y" corresponds to the machine direction (MD), and the transverse axis "x" corresponds to the cross direction (CD), relative to the orientation of the manufacturing line. The absorbent assembly (1) comprises two opposing longitudinal edges (5a, 5a') and two opposing lateral edges (6a, 6a'). The two opposing longitudinal edges (5a, 5a') extend along the direction of the longitudinal axis "y" of the absorbent assembly (1). The two opposing lateral edges (6a, 6a') extend along the direction of the transversal axis "x" of the absorbent assembly (1). The absorbent assembly (1) comprises a front region (21), oriented toward the belly of the wearer, a back region (23), oriented toward the back of the wearer and a crotch region (22) positioned between the front region and the back region. The longitudinal and lateral edges of the absorbent assembly (1) may be defined at least in part by the upper fluid-permeable layer (20), the lower fluid-impermeable layer (30) or both. In some embodiments, the absorbent assembly (1) may comprise bonded regions of the upper fluid-permeable layer (20) and the lower fluid-impermeable layer (30), wherein said bonded regions are either continuous or discontinuous.

In one embodiment, the at least one layer of material that connects the first and second absorbent assemblies (1a, 1b) may be the upper fluid-permeable layer (20), the lower fluid-impermeable layer (30), or both. For example, the upper fluid permeable layer (20) may extend substantially continuously in the longitudinal direction of the band of absorbent assemblies (12), such that adjacent first and second absorbent assemblies (1a, 1b) both comprise at least a portion of the same upper fluid permeable layer (20). In another example, the lower fluid-impermeable layer (30) may extend substantially continuously in the longitudinal direction of the band of absorbent assemblies (12), such that adjacent first and second absorbent assemblies (1a, 1b) both comprise a portion of the same lower fluid-impermeable layer (30). In some embodiments, both the upper fluid permeable layer (20) and the lower fluid-impermeable layer (30) may extend substantially continuously in the longitudinal direction of the band of absorbent assemblies (12), as described. In some embodiments one or more materials in addition to or in the alternative to the upper fluid permeable layer (20) and the lower fluid-impermeable layer (30) may extend substantially continuously in the longitudinal direction of the band of absorbent assemblies (12), to connect the first and second absorbent assemblies (1a, 1b).

In some embodiments, the adjacent first and second absorbent assemblies (1a, 1b) may be coupled with one or more discontinuous layers of material. The one or more discontinuous layers of material may be in addition to or an alternative to a material layer that extends substantially continuously in the longitudinal direction of the band of absorbent assemblies (12). For example, a discontinuous layer of material may extend between and be coupled to the back region (23) of the first absorbent assembly (1a) and the front region (21) of the adjacent second absorbent assembly (1b). In this way the at least one discontinuous layer connects adjacent first and second absorbent assemblies (1a, 1b). An exemplary discontinuous layer of material could be a separate nonwoven layer, film layer, a waist band element, a fastening element, or the like.

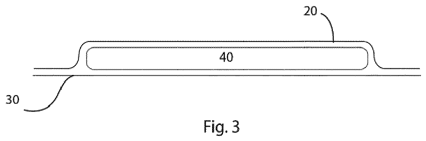

Fig. 3 shows a cross-sectional view of the (X-X) section of the non-limiting embodiment of an absorbent assembly (1) of Fig. 2. The absorbent assembly (1) has an upper fluid-permeable layer (20) oriented toward the skin of the wearer and a lower fluid-impermeable layer (30) oriented toward the garment of the wearer.

The absorbent assembly (1) may optionally comprise one or more other elements as necessary or desired to form a disposable absorbent article. For example, the optional elements may be include a liquid management system, a closure system, an antileakage system, a disposal system, an attachment system, or the like.

In some embodiments, each of the absorbent assemblies (1) may form a disposable absorbent article such as a taped diaper, refastenable pants, sanitary napkins, pads, inserts or combinations thereof.

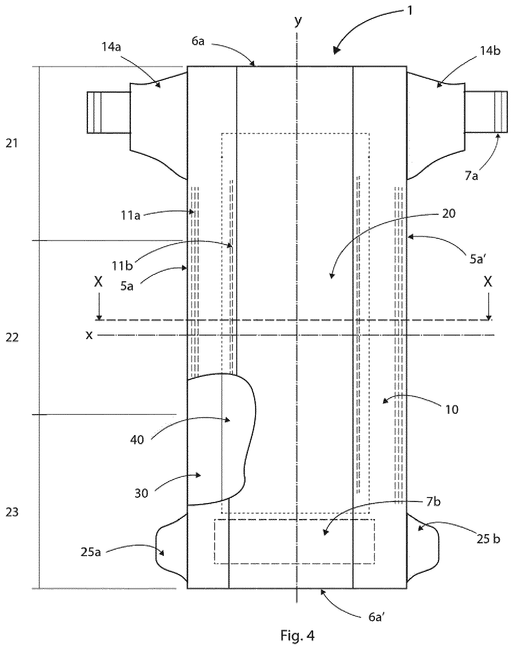

For example, referring to FIG. 4, the absorbent assembly (1) may be in the form of a taped diaper. In an exemplary taped diaper, the absorbent assembly (1) may have a closure system (7a, 7b). The closure system may be a mechanical closure system (e.g. hook and loop or hook and backsheet), adhesive closure system, or combinations thereof. The closure system may be joined to side panels, belts, ears, tabs or the like to enable the absorbent article to be worn around the waist. Referring to FIG. 4, in certain embodiments, a portion of the closure system (7a) is attached to side panels (14a, 14b) disposed adjacent the front and/or back region. The side panels (14a, 14b) may be discrete pieces made of a single layer of material or multiple layers of material such as nonwoven, film or combinations thereof. In some embodiments, the side panels (14a, 14b) may be a laminate with at least one elastic element. The elastic elements of said side panels may be elastic threads, bands, films, perforated films, elastic nonwoven or combinations thereof. The side panels (14a, 14b) may be joined to the front region (21), back region (23) or both regions of the main structure. In some embodiments, the absorbent assembly may have frontal ears (25a, 25b), which may be the same as or different from side panels (14a, 14b). The frontal ears may be discrete pieces of elastic or inelastic material such as nonwoven, film or combinations thereof.

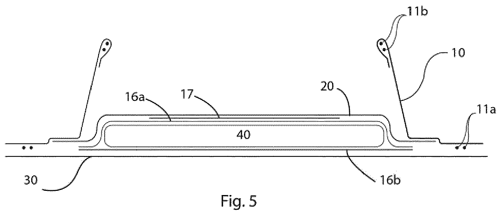

As seen in Fig. 5, other layers or elements may optionally be positioned between the absorbent core (40), the upper fluid-permeable layer (20) and the lower-fluid impermeable layer (30) such as an acquisition-distribution layer (17), single core wrap layer, upper and lower core wrap layers (16a, 16b) or combinations thereof.

The taped diaper may optionally comprise one or more other elements or layers to avoid leakage. The elements or layers to avoid leakage may be frontal antileakage barrier, back antileakage barrier, leg antileakage barriers or the like. Said antileakage barriers (10) may comprise one or more elastic elements (11b). The one or more elastic elements may be elastic strands, ribbons, nonwoven, film, perforated film, foam or any other elastic material known in the art.

The taped diaper may optionally include elastic elements disposed in the main structure, such as leg elastics (11a), waist elastics or combinations thereof. The leg elastics (11a) may extend at least through the crotch region, in a longitudinal direction and adjacent to the longitudinal edges of the absorbent article. The waist elastics may extend at least one of the front and back regions of the disposable absorbent article, in a transversal direction and adjacent to at least one of the lateral edges of the absorbent article. The leg and waist elastics may be elastic strands, ribbons, nonwoven, film, perforated film, foam or any other elastic material known in the art.

One having ordinary skill in the art would understand these and additional features and structures that an absorbent assembly (1) would have when in the form of a taped diaper.

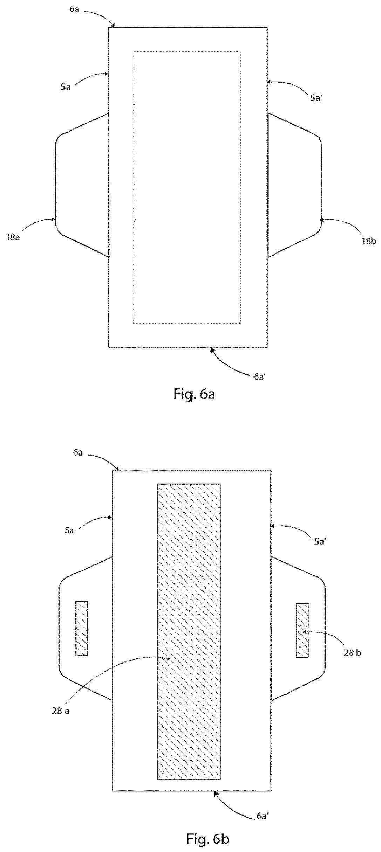

In other embodiments, the absorbent assembly (1) may be in the form of a feminine hygiene product, such as a sanitary napkin, as seen in Figs. 6a and 6b. In such articles, the absorbent assembly may optionally comprise wings (18a, 18b) to enable the disposable absorbent article to be attached to the clothes of the wearer. In certain embodiments, the wings may be discrete or continuous portions of either the upper fluid-permeable layer (20) or the lower fluid-impermeable layer (30). The wings (18a, 18b) may be bonded to the front region (21), the crotch region (22), the back region (23) of the main structure of the disposable absorbent article or combinations thereof. The absorbent assembly may optionally comprise at least one adhesive layer (28a) in the lower (garment-facing) surface so as to enable the disposable absorbent article to be attached to the clothes of the wearer. The wings may also comprise an adhesive layer (28b) for fixing the absorbent assembly to the clothes of the wearer. One having ordinary skill in the art would understand these and additional features and structures that an absorbent assembly (1) would have when in in the form of a feminine hygiene article.

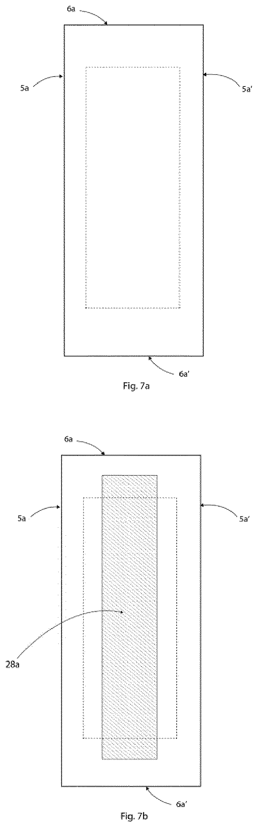

In other embodiments, the absorbent assembly (1) may be in the form of an absorbent insert, as seen in Figs. 7a and 7b. An exemplary absorbent insert may be used in combination with a disposable or reusable article, such as a reusable outer cover, or reusable garment. In such articles, the absorbent assembly may optionally comprise a fastener component such as an adhesive layer (28a) applied on at least a portion of the external surface of the lower fluid-impermeable layer (30) to enable it to be attached to the outer cover or garment. The fastener component may alternatively or additionally include mechanical attachment elements (e.g. hook/loop) to enable it to be attached to the outer cover or garment. As desired, the absorbent insert may include one or more of the features described herein with respect to the other embodiments, such as, for example, leg elastics.

The band (12) of absorbent assemblies (1a, 1b, ... 1n) includes a plurality of longitudinally spaced weakened division lines (13). Weakened division lines (13) are disposed between each absorbent assembly (1a, 1b, ... 1n) in the band (12), for example between the absorbent core (40) of the first absorbent assembly (1a) and the absorbent core (40) of the second absorbent assembly (1b). The weakened division lines (13) help to separate the first absorbent assembly (1a) from the second absorbent assembly (1b) when required or desired by the manufacturer or the consumer. Weakened division lines (13) are applied to the band (12) of absorbent assemblies (1a, 1b, ... 1n) with means for applying weakened division lines (12). For example, weakened division lines (13) can be applied by cutting, perforating, stretching, scratching such as with the use of a die cutter or a knife cutter, and the like. Weakened division lines (13) can also be applied by chemical treatment such as the application of acid or thermal treatment such as a heating, partial burning or freezing. The weakened division lines (13) are preferably applied in a cross direction perpendicular to the machine direction. The weakened division lines (13) may be spaced on the band (12) of absorbent assemblies (1a, 1b ... 1n) at regularly spaced intervals along the machine direction.

The weakened division line (13) provides a region that has a reduced strength as compared to other regions of the band (12). For example, the weakened division line (13) may provide a reduced tensile strength or a reduced tear strength relative to other regions of the band. As such, when a separation force is applied to the band (12), it will preferentially break or separate at or adjacent the weakened division line (13). However, the weakened division line (13) has sufficient strength so that the band (13) can be manufactured and handled as desired without breaking and tearing. According to various embodiments, the weakened division line (13) is generally oriented in the cross direction of the band.

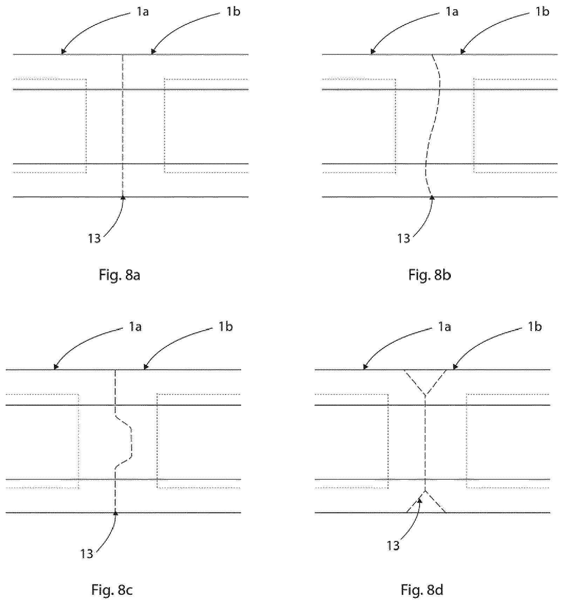

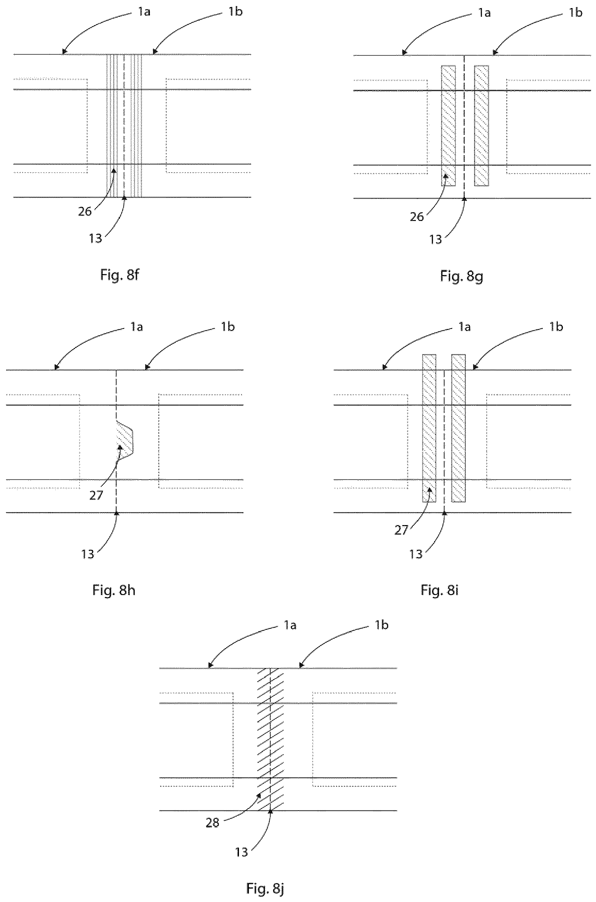

According to some aspects of the invention, the weakened division line (13) may have a specific shape. For example, the weakened division line (13) may include a linear shape, a curvilinear shape, an undulating shape, an umbilical cut-out shape, or a combination thereof, as exemplified in Figs. 8a - 8c. In further examples of the present invention, the weakened division line (13) may provide a cut-out shape for providing a different final shape for the absorbent assembly, as shown in Fig. 8d.

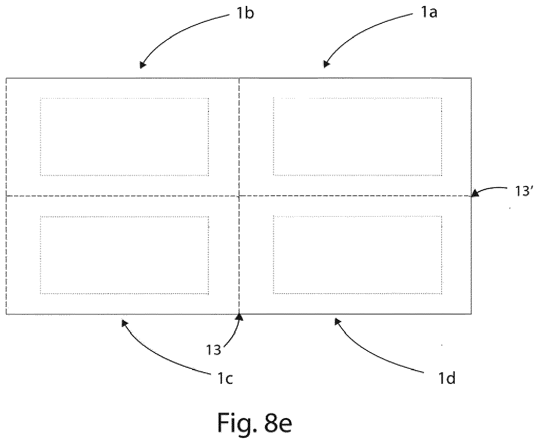

In some embodiments, a band (12) may have one or more additional weakened division lines. For example, referring to FIG. 8e, a band (12) may be configured to have adjacent absorbent assemblies in the machine direction (absorbent assemblies (1a) and (1b) are adjacent in the machine direction, as are absorbent assemblies (1c) and (1d)) and in the cross direction (absorbent assemblies (1a) and (1c) are adjacent in the cross direction, as are absorbent assemblies (1b) and (1d)). In this type of configuration, a first weakened division line (13) may be provided between absorbent assemblies (1a, 1b) that are adjacent in the machine direction, and an additional weakened division line (13') may be provided between absorbent assemblies (1a, 1c), that are adjacent in the cross direction.

According to some aspects, the band (12) may include a reinforcing means (26) to increase or control the tensile strength or tear strength of the weakened division line (13). For example, a reinforcing layer or additional bonding may be provided at or adjacent to the weakened division line (13), as shown in Figs. 8f - 8g. According to some aspects, the band may include one or more separation mechanisms (27) to assist or control the separation of the band (12) at the weakened division line (13). For example, a finger lift or tear strip can be provided to initiate separation at the weakened division line (13), as shown in Figs. 8h - 8i. According to some aspects, the band (12) may include one or more visual or tactile aids (28) to help identify the weakened division line (13). For example, a printed guideline or embossed guide may be provided at or near the weakened division line (13), as shown in Fig. 8j.

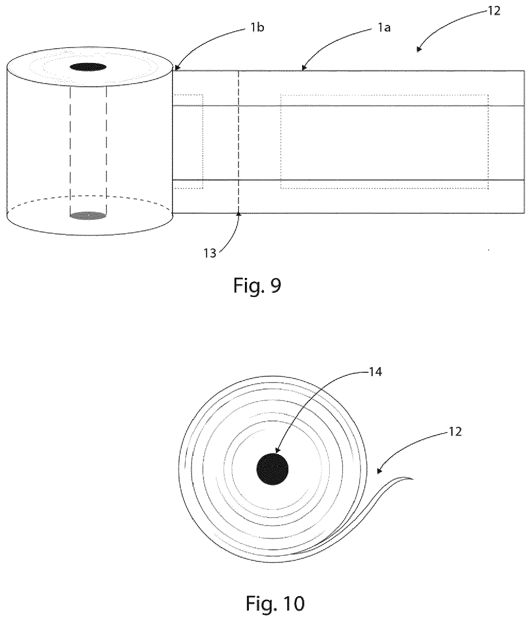

In some aspects of the invention, once the band of absorbent assemblies is formed, it is rolled or spooled into a cylindrical configuration. Fig. 9 shows a perspective view of an exemplary band of absorbent assemblies that has been rolled to form a cylindrical shape. In an embodiment, the band of absorbent assemblies may be rolled about a core. The core may be a cylinder, a tube or the like. In further embodiments, the band of absorbent assemblies may be rolled without a cylinder or a tube, in a core-less configuration. The cylinder or tube may be made of cardboard or plastic such as the cylinders used for paper tissue articles. The rolled band of absorbent assemblies enables the manufacturer and the consumer to remove more easily at least one disposable absorbent articles, depending on the required quantity.

The process for rolling the band of absorbent assemblies includes a step of engaging a free edge of said band of disposable absorbent assemblies on a winding shaft. Once the free edge is engaged, the winding shaft may rotate so as to coil said band of absorbent assemblies and thereby form a roll or spool. Suitable methods for rolling or spooling the band of disposable absorbent articles of the present invention are disclosed in the following U.S. patent No. 4,487,378 or US Publication No. 2017/0037579, which are incorporated herein by reference in their entirety. In one embodiment, when rotating the band of absorbent assemblies, the longitudinal axis of said band of absorbent assemblies may be aligned with the central axis of the winding shaft. In an alternative embodiment, when rotating the band of absorbent assemblies, the longitudinal axis of said band of absorbent assemblies may form an acute angle from the central axis of the winding shaft. Said acute angle may be between 5° to 45°, between 8° to 30°, between 10° to 25°, between 15° to 20°.

Fig. 10 shows a top view of an exemplary band of disposable absorbent assemblies disposed in cylindrical shape, according to one embodiment. The cylindrical configuration of the band of disposable absorbent assemblies allow the finished product to be compressed even more than conventional packages of disposable absorbent articles and therefore to occupy a smaller volume during distribution and storage of the product. In one embodiment, the body-facing surface of the disposable absorbent article is oriented to face the core of the rolled band of disposable absorbent articles, thus protecting the surface of the disposable absorbent article destined to be in contact with the skin of the user prior to its use.

In further embodiments, there may be a constant thickness through the entire length of the absorbent assemblies so that there is a consistent diameter in the cylindrical configuration of the band of disposable absorbent assemblies.

In absorbent assemblies comprising elastic elements, such as leg elastics, the elastic elements that may be used in the absorbent assemblies have between 50% to 100% of elastic recovery, or between 60% to 100% of elastic recovery, or between 70% to 100% or elastic recovery, or between 80% and 100% of elastic recovery after being submitted to a deforming force resulting from the rolling the band of disposable absorbent assemblies in a cylinder configuration. The elastic recovery of the elastic elements helps the absorbent article to have sufficient elastic properties during its use, especially to maintain improved fit and antileakage properties. An exemplary method for measuring the elastic recovery is described below.

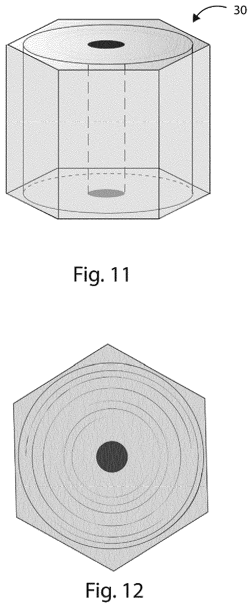

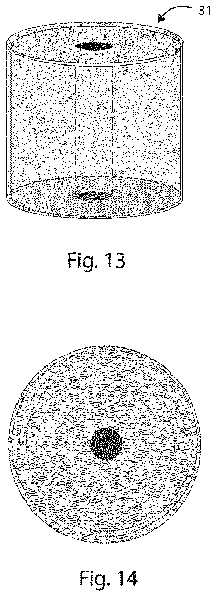

In certain embodiments, the rolled band of disposable absorbent assemblies may be packaged in a container. For example, as seen in Figs. 11 and 12, the rolled band of disposable absorbent assemblies may be packaged in a box or prism (30). The box or prism may be circular, triangular, rectangular, pentagonal, hexagonal or the like. The box or prism may be at least partially rigid. The prism may be made of cardboard, flexible polymeric material or combinations thereof. In further embodiments, as seen in Figs. 13 and 14, the rolled band of disposable absorbent articles may be packaged within a flexible cover (31), to protect the exterior surface before its use, the flexible cover may comprise film from renewable or petrochemical sources, paper or combinations thereof. In an embodiment, the rolled band of disposable absorbent articles may be packaged with a siliconized paper to protect the adhesive layer used to attach the disposable absorbent article to the garment of the user.

It may be desirable that the rolled band of absorbent assemblies is compressible. The compression of the material contributes directly to decrease the volume of the rolled band of absorbent assemblies, and consequently contributes to improve the storage and distribution of the finished products. Additionally, the decreased volume of the rolled band of absorbent assemblies requires less packaging material to cover the entire product.

In an embodiment, at least one of the upper fluid-permeable layer and the lower fluid-impermeable layer comprise a percentage of compression of at least 15%, or at least 17%, or at least 20%, or at least 25%, or at least 30% when measured according to the Compression Test described herein. For example, the upper fluid permeable layer, the lower fluid impermeable layer or both may have a percentage compression of between 15% and 90%, between 17% and 80%, between 20% and 70%, between 25% and 60%, or between 30% and 50%, according to the Compression Test described herein.

In further embodiments, other layers intermediate to the upper fluid-permeable layer and the lower fluid-impermeable layer, including the absorbent core, may have a compression of at least 15% or at least 17% or at least 20% or at least 25% or at least 30%, according to the Compression Test. For example, one or more of the layers intermediate to the upper fluid-permeable layer and the lower fluid-impermeable layer, including the absorbent core, may have a percent compression of between 15% and 90%, between 17% and 80%, between 20% and 70%, between 25% and 60%, between 30% and 50%, according to the Compression Test.

It may be desirable that an absorbent assembly, when separated from the rolled band of absorbent assemblies, substantially recovers the original calliper of the absorbent assembly, i.e., the calliper prior to spooling the band of absorbent assemblies.

In an embodiment, at least one of the upper fluid-permeable layer and the lower fluid-impermeable layer may comprise a compression set of 80% at most, or 70% at most, or 60% at most, or 50% at most, according to the Compression test described herein. A lower compression set indicates an improved restoration of the original properties of the layer or layers of materials after being submitted to a compressive force such as the force on the absorbent assembly in the roll or spool; in the case of a rolled band of disposable absorbent assemblies, the disposable absorbent assemblies, tightly compressed due to the force applied to roll the band of disposable absorbent articles in a cylindrical shape, may substantially restore the original calliper once unwound and/or individually removed from the rolled good.

In an embodiment, each absorbent assembly in the band of absorbent assemblies of the present invention may comprise bio-based materials. For example, an absorbent assembly may comprise at least 10% by weight of bio-based materials, at least 20% of bio-based materials, 30% of bio-based materials, at least 40% of bio-based materials, at least 50% of bio-based materials, at least 60% of bio-based materials, at least 60% of bio-based materials, at least 70% of bio-based materials, at least 80% of bio-based materials or at least 90% of bio-based materials. Available techniques to measure the content of bio-based materials are known in the art, for example, the carbon-14 dating method detailed below.

**Elastic Recovery Test**

Specimen Preparation:

1. The disposable absorbent article is removed from the container where it has been packaged and placed in a flat surface.
2. Identify the elastic elements placement within the disposable absorbent article.
3. Separate the elastic elements from the disposable absorbent article by means of cryogenic spray or acetone, depending on the amount of adhesive used to laminate the finished product, so that the elastic elements remain substantially intact.
4. Let the elastic elements rest for 5 minutes.

Test method:

1. Measure the length of an individual elastic element and record it as Original Length ($L_0$).
2. Stretching the elastic elements at 100% of elongation for 30 seconds.
3. Measure the length of the elastic element and record it as Stretched Length ($L_s$)
4. Let the elastic elements rest for 5 minutes.
5. After 5 minutes, measure the length of the elastic element and record it as Relaxed length ($L_r$).
6. Repeat with 5 samples in total.

Elastic recovery is measured with the following equation:
Elastic recovery %= $(L_s - L_r)/L_0*100$, where $L_s$ is the stretched length of the elastic, $L_r$ is the length of the elastic after relaxation and $L_0$ is the original length.

**Compression test**

Specimen Preparation:

1. The disposable absorbent article is removed from the container it has been packaged and extended on a plain surface, with the upper/lower surface of interest oriented upwards.
2. Remove the upper/lower layer or interest from the main structure. If necessary, use acetone or a cryogenic spray, depending on the amount of adhesive used to laminate the finished product.
3. Cut square samples from the removed layer (60 mm * 60 mm each). Preferably cut the samples in the central region of the layer of material.

Test method:

Compression measurements are performed using Instron Universal testing system (Instron 5969 or equivalent) capable of measuring from 0.02 N (2 gf) up to 50 kN. The equipment comprises a circular foot with a diameter of about 50.8 mm. The equipment is tared by placing the circular foot directly on the anvil and setting the digital gauge to a zero value. The circular foot is raised enough to allow the sample of material to be placed on the caliper anvil, with the first test sample placed under the foot. The foot is lowered at a speed of 5 mm/sec and allowed to rest on the sample, applying a pressure of 0.6 psi for 30 seconds and then raised again until no pressure is applied to the sample.

Readings are taken as follows:

- Immediately when the foot touches the sample, recorded to the nearest 0.01 mm and reported as the initial calliper.
- After a residence time of 30 seconds with a pressure of 0.6 psi, recorded to the nearest 0.01 mm and reported as the compressed calliper.
- Immediately after the foot is raised and no pressure is applied, recorded to the nearest 0.01 mm and reported as decompressed calliper.

The same steps and measurement are repeated on the second sample. A total often substantially identical samples are analysed and the average of all the measurements of the calliper reported to the nearest 0.01 mm.

The Compression % is calculated as [(initial calliper) − (compressed calliper)]/(initial calliper)×100%

The Compression set is calculated as [(initial calliper) − (decompressed calliper)]/(initial calliper)-(compressed calliper)×100%

**Carbon-14 test**

The Carbon-14 test, also referred to as radiocarbon test, may be used to determine the amount of bio-based material present in a finished product or raw material. Research has shown that this technique distinguishes fossil matter from living matter in view of the amount of Isotope C14 present in them. Isotope C14 occurs naturally in all living organisms in a fixed concentration of $1.2 \times 10^{-12}\%$, while, in fossil sources, the isotope C14 will be present in a much lower concentration, $1.2 \times 10^{-15}\%$ or less since the isotope is no longer acquired by dead matter and the isotope concentration decays during the years. Some techniques for carbon-14 analysis include accelerator mass spectrometry, liquid scintillation counting, and isotope mass spectrometry, said techniques can be implemented either in the complete end product or in raw material. Examples of standard tests include NIST 4990C, ASTM D6866, ISO 16620-2 and EN 16640.

In the preceding specification, various embodiments have been described with reference to the accompanying drawings. It will, however, be evident that various modifications and changes may be made thereto, and additional embodiments may be implemented, without departing from the broader scope of the exemplary embodiments as set forth in the claims that follow. The specification and drawings are accordingly to be regarded in an illustrative rather than restrictive sense.

**Claims**

1. A band of disposable absorbent assemblies having at least first and second disposable absorbent assemblies, wherein each of the first and second disposable absorbent assemblies comprises an absorbent core interposed between an upper fluid-permeable layer and a lower fluid-impermeable layer; wherein the first and the second disposable absorbent assemblies are connected by at least one layer of material; wherein a weakened division line is disposed between the absorbent core of the first disposable absorbent assembly and the absorbent core of the second disposable absorbent assembly; wherein the band of disposable absorbent assemblies is rolled into a cylindrical shape; and wherein at least one of the upper fluid-permeable layer and the lower fluid-impermeable layer has a compression set of 80% or less, according to the Compression test described herein.

2. The band of disposable absorbent assemblies according to claim 1, wherein at least one of the upper fluid-permeable layer, the lower fluid-impermeable layer, and an intermediate layer between the upper fluid-permeable layer and the lower fluid-impermeable layer has a percentage of compression of at least 15%, according to the Compression test described herein.

3. The band of disposable absorbent assemblies according to any of the previous claims, wherein the at least one layer of material connecting the at least first and second absorbent assemblies comprises a continuous material, optionally at least one of the upper fluid-permeable layer and the lower fluid-impermeable layer.

4. The band of disposable absorbent assemblies according to claim 1, wherein the at least one layer of material connecting the first and second disposable absorbent assemblies comprises a discontinuous material, optionally at least one of nonwoven layer, film layer, waist band element and fastening element.

5. The band of disposable absorbent assemblies according to any of the previous claims, wherein the weakened division line is formed by one or more of a mechanical, thermal, and chemical process, or combinations thereof; optionally wherein the weakened division line has a linear shape, a curvilinear shape, an undulating shape, an umbilical cut-out shape, or a combination thereof.

6. The band of disposable absorbent assemblies according to any of the previous claims, wherein the band comprises one or more reinforcing means adjacent to the weakened division line, optionally wherein the reinforcing means comprises a reinforcing layer, additional bonding element, or combinations thereof.

7. The band of disposable absorbent assemblies according to any of the previous claims, wherein the band comprises one or more separation mechanisms, optionally wherein the one or more separation mechanisms comprise a finger lift, a tear strip, or a combination thereof; or one or more visual aids, optionally wherein the visual aids comprise a printed guideline, an embossed guide, or combination thereof; or a combination thereof.

8. The band of disposable absorbent assemblies according to any of the previous claims, wherein the band has one or more additional weakened division lines.

9. The band of disposable absorbent assemblies according to any of the previous claims, wherein said band of disposable absorbent assemblies is rolled about a cylindrical or tubular core or is rolled in a core-less configuration.

10. The band of disposable absorbent assemblies according to any of the previous claims, wherein each of the absorbent assemblies has one or more elastic elements and the elastic elements have at least 50% of elastic recovery, according to the Elastic Recovery Test.

11. The band of disposable absorbent assemblies according to any of the previous claims, wherein the band is disposed in a package, wherein the package is a box or prism, optionally wherein the package is at least partially rigid, optionally wherein the package comprises cardboard, flexible polymeric material or combinations thereof.

12. The band of disposable absorbent assemblies according to any of the previous claims, wherein the package comprises a flexible cover comprising film from a renewable source, film from a petrochemical source, paper, siliconized paper or combinations thereof.

13. The band of disposable absorbent assemblies according to any of the previous claims, wherein each of the at least first or second absorbent assemblies comprises an adhesive layer that is disposed on a garment-facing surface of the lower fluid impermeable layer, optionally wherein each of the at least first or second absorbent assemblies comprises a siliconized paper layer disposed on the adhesive layer.

14. A band of disposable absorbent assemblies having at least first and second disposable absorbent assemblies, wherein each of the first and second disposable absorbent assemblies comprises an absorbent core interposed between an upper fluid-permeable layer and a lower fluid-impermeable layer; wherein the first and the second disposable absorbent assemblies are connected by at least one layer of material; wherein a weakened division line is disposed between the absorbent core of the first disposable absorbent assembly and the absorbent core of the second disposable absorbent assembly; wherein the band of disposable absorbent assemblies is rolled into a cylindrical shape; and wherein each of the at least first or second disposable absorbent assemblies comprises at least 10% by weight of biobased material.

15. A disposable absorbent assembly comprising the first or second disposable assembly of the band of disposable absorbent assemblies according to any of the previous claims, wherein the disposable absorbent assembly is a taped diaper, a sanitary napkin, or a detachable absorbent insert.

MD

1a
1b
12

13

Fig. 1a

MD
40
1a
13
20
1b
40
20
30
30

Fig. 1b

Fig. 2

Fig. 3

Fig. 4

Fig. 5

Fig. 6a

Fig. 6b

6a

5a 5a'

6a'

Fig. 7a

6a

5a 5a'

28a

6a'

Fig. 7b

1a    1b

13

Fig. 8a

1a    1b

13

Fig. 8b

1a    1b

13

Fig. 8c

1a    1b

13

Fig. 8d

Fig. 8e

Fig. 8f

Fig. 8g

Fig. 8h

Fig. 8i

Fig. 8j

Fig. 9

Fig. 10

Fig. 11

Fig. 12

Fig. 13

Fig. 14

Europäisches
Patentamt

European
Patent Office

Office européen
des brevets

# EUROPEAN SEARCH REPORT

**Application Number**

EP 23 15 6655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/069032 A1 (DEVLIN THOMAS E [US]) 11 March 2021 (2021-03-11) | 1-5, 7-10, 13-15 | INV.<br>A61F13/47<br>A61F13/49<br>A61F13/551 |
| Y | * paragraph [0008] * | 11,12 | |
| A | * paragraph [0011] *<br>* paragraph [0087] – paragraph [0092] *<br>* paragraph [0100] – paragraph [0102] *<br>* paragraph [0105] *<br>* paragraph [0111] – paragraph [0112] *<br>* paragraph [0118]; figures 1, 2-2C, 3-3B, 5-5C, 6-6B, 10-10B * | 6 | |
| | ----- | | |
| X | US 4 598 528 A (MCFARLAND TIMOTHY M [US] ET AL) 8 July 1986 (1986-07-08) | 1-5, 7-12,14, 15 | |
| Y | * column 4, line 1 – line 22 * | 11,12 | |
| A | * column 5, line 3 – line 45 *<br>* column 5, line 52 – column 6, line 21 *<br>* column 7, line 34 – line 52; figures 1-7, 10, 12-15 * | 6,13 | |
| | ----- | | |
| X | US 6 254 582 B1 (O'DONNELL KATHLEEN DENISE [US] ET AL) 3 July 2001 (2001-07-03)<br>* column 1, line 47 – line 50 *<br>* column 2, line 18 – line 37 *<br>* column 3, line 60 – column 4, line 38 *<br>* column 5, line 5 – line 19; figures 1-5 * | 1,14,15 | **TECHNICAL FIELDS SEARCHED (IPC)**<br><br>A61F |
| | ----- | | |
| A | US 3 183 910 A (PATTERSON LLOYD D) 18 May 1965 (1965-05-18)<br>* the whole document * | 1-15 | |
| | ----- | | |
| A | US 4 505 704 A (ROEDER ROBERT J [US]) 19 March 1985 (1985-03-19)<br>* the whole document * | 1-15 | |
| | ----- | | |

The present search report has been drawn up for all claims

| Place of search | Date of completion of the search | Examiner |
|---|---|---|
| Munich | 13 June 2023 | Demay, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 15 6655**

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2023

| Patent document cited in search report | | Publication date | Patent family member(s) | | Publication date |
|---|---|---|---|---|---|
| US 2021069032 | A1 | 11-03-2021 | NONE | | |
| US 4598528 | A | 08-07-1986 | CA | 1261795 A | 26-09-1989 |
| | | | US | 4598528 A | 08-07-1986 |
| US 6254582 | B1 | 03-07-2001 | AT | 165507 T | 15-05-1998 |
| | | | AU | 685431 B2 | 22-01-1998 |
| | | | BR | 9405660 A | 21-11-1995 |
| | | | CA | 2154176 A1 | 04-08-1994 |
| | | | CN | 1116821 A | 14-02-1996 |
| | | | DE | 69409941 T2 | 17-12-1998 |
| | | | EP | 0680304 A1 | 08-11-1995 |
| | | | ES | 2115938 T3 | 01-07-1998 |
| | | | FI | 953512 A | 20-07-1995 |
| | | | JP | H08505800 A | 25-06-1996 |
| | | | NO | 304053 B1 | 19-10-1998 |
| | | | NZ | 262251 A | 28-10-1996 |
| | | | PL | 309643 A1 | 30-10-1995 |
| | | | TW | 274512 B | 21-04-1996 |
| | | | US | 6254582 B1 | 03-07-2001 |
| | | | WO | 9416659 A1 | 04-08-1994 |
| | | | ZA | 94394 B | 20-07-1995 |
| US 3183910 | A | 18-05-1965 | NONE | | |
| US 4505704 | A | 19-03-1985 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4487378 A

- US 20170037579 A